# EUROPÄISCHE PATENTSCHRIFT

(11) **EP 1 752 119 B1**
(45) Veröffentlichungstag und Bekanntmachung des Hinweises auf die Patenterteilung: **15.10.2008**
(21) Anmeldenummer: 05017648.6
(22) Anmeldetag: 12.08.2005
(51) Int. Cl.: A61F 9/013

(54) **Schneidklingenhalter für eine mikrochirurgische Schneidanordnung, insbesondere eine solche für die refraktive Augenchirurgie**
Blade holder for microsurgical cutting assembly, in particular for eye surgery
Porte-lame pour un dispositif de coupe microchirurgical, en particulier pour chirurgie oculaire

(43) Veröffentlichungstag der Anmeldung: 14.02.2007
(73) Patentinhaber: WaveLight Laser Technologie AG, 91058 Erlangen (DE)
(72) Erfinder: Donitzky, Christof, 90542 Eckental (DE); Thimm, Daniel, 90596 Schwanstetten (DE)
(74) Vertreter: von Hellfeld, Axel

(56) Entgegenhaltungen:
- US-A- 5 989 272
- US-A1- 2004 186 494
- US-B1- 6 254 619
- US-B1- 6 610 075

## Beschreibung

Die Erfindung betrifft einen Schneidklingenhalter für eine mikrochirurgische Schneidanordnung, insbesondere eine Schneidanordnung für die refraktive Augenchirurgie, wobei der Schneidklingenhalter eine Aufnahme aufweist, in die eine Schneidklingeneinheit mit einer Schneidklinge einsetzbar ist, wobei weiter der Schneidklingenhalter Führungsmittel zur seitbeweglichen Führung der Schneidklingeneinheit in der Aufnahme umfasst.

Zur refraktiven Sehfehlerbehandlung des menschlichen Auges ist es bekannt, mittels eines als Mikrokeratom bezeichneten mikrochirurgischen Schneidinstruments ein oberflächliches Scheibchen (Flap) von der Hornhaut so zu trennen, dass es an einem sogenannten Hinge noch mit der Hornhaut verbunden ist. Durch Anheben und Wegklappen des Flaps werden die darunterliegenden Hornhautbereiche (Stroma) zur Hornhautneuformung mittels eines Lasers zugänglich. Nach Beendigung der Laserbehandlung wird der Flap zurückgeklappt.

Das Mikrokeratom weist üblicherweise eine auf den Augapfel (Limbus) aufzusetzende Saugringeinheit auf, an der ein mit einer Schneidklinge auswechselbar bestückter Schneidklingenhalter beweglich geführt ist. Zur Flappräparation wird der Schneidklingenhalter mittels eines elektromotorischen Antriebs in einer Vorschubrichtung über die Hornhaut bewegt. Dabei schneidet die mit ihrer vorderen Schneidkante aus dem Schneidklingenhalter vorstehende Schneidklinge in die Hornhaut ein und löst den Flap ab.

Zusätzlich zum Vorschub des Schneidklingenhalters wird üblicherweise die Schneidklinge in laterale Oszillation versetzt. Oszillationsfrequenzen zwischen 15 und 500 Hz, insbesondere zwischen 100 und 250 Hz sind hierbei nicht unüblich. Bei diesen hohen Frequenzen der seitlichen Hin- und Herbewegung der Schneidklinge sollten die in dem Schneidklingenhalter vorgesehenen Führungsmittel eine leichtgängige und abriebarme, jedoch nichtsdestoweniger präzise Führung einer die Schneidklinge aufweisenden Schneidklingeneinheit in dem Schneidklingenhalter gewährleisten können.

Dokument US 5989272 offenbart einen Schneidklingenhalter, der zusätzlich zu zwei Führungsflächen einen wahlweise in die eine oder die andere Führungsfläche eingelassenen Führungsstab aufweist.

Diesbezüglich umfassen bei einem Schneidklingenhalter der eingangs bezeichneten gattungsgemäßen Art erfindungsgemäß die Führungsmittel beidseits der Klingenebene der Schneidklinge angeordnete Führungsformationen, welche näherungsweise Punkt- oder Linienkontakt mit der Schneidklinge herstellen. Infolge der unmittelbaren Führung der Flachseiten der Schneidklinge an den Führungsmitteln kann eine hohe Führungspräzision der Klinge erzielt werden. Der Punkt- oder Linienkontakt der Klinge mit den Führungsmitteln sorgt hierbei für eine geringe Reibung und entsprechend auch einen geringen Verschleiß, was sich wiederum günstig auf die Konstanz der speziell bei Augenoperationen regelmäßig erforderlichen äußerst hohen Schneidgenauigkeit auswirkt. Wenn hier von einem Punkt- oder Linienkontakt die Rede ist, so versteht es sich, dass aufgrund von Mikroverformungen der einander kontaktierenden Bauteile und aufgrund niemals perfekt ideal glatter Oberflächen dieser Bauteile nicht im streng mathematischen Sinne ein Punkt- oder Linienkontakt zwischen der Schneidklinge und den Führungsformationen herrschen wird, sondern nur im technischen Sinne.

Bei einer Ausführungsform ist mindestens eine Führungsformation von einem in Klingenquerrichtung sich erstreckenden, an einem Grundkörper des Schneidklingenhalters gehaltenen Führungsstab gebildet. Insgesamt können ohne weiteres mehrere solcher Führungsstäbe ober- oder/und unterhalb der Schneidklinge in der Aufnahme vorgesehen sein.

Mehrere Führungsformationen können bei Bedarf auch einstückig an einem gemeinsamen Führungskörper des Schneidklingenhalters ausgebildet sein. Beispielsweise könnte in die Aufnahme mindestens eine Führungsplatine eingefügt sein, welche an ihrer der Schneidklinge zugewandten Seite eine Anordnung von lokal vorstehenden Führungsnoppen trägt, die für sich jeweils näherungsweise Punktkontakt mit der Schneidklinge herstellen. Alternativ könnten mehrere längliche Führungsrippen, die jeweils einen Linienkontakt mit der Schneidklinge herstellen, an einem gemeinsamen Führungskörper ausgebildet sein, wobei dieser Führungskörper ein von einem Grundkörper des Schneidklingenhalters gesondert hergestelltes Bauteil sein kann oder unmittelbar von einem solchen Grundkörper gebildet sein kann.

Zur Erzielung eines Punkt- oder Linienkontakts kann mindestens eine Führungsformation bei Betrachtung in einem Schnitt quer zur Klingenquerrichtung eine bogenartig gekrümmte oder alternativ eine spitz zulaufende Kontur im Bereich einer Führungsanlagestelle besitzen.

Die Führungsformationen können so verteilt sein, dass mindestens ein Paar von beidseits der Klingenebene angeordneten Führungsformationen einander bei Betrachtung in einem Schnitt quer zur Klingenquerrichtung zumindest näherungsweise gegenüberliegt. Es ist aber auch denkbar, dass mehrere Führungsformationen bei Betrachtung in einem Schnitt quer zur Klingenquerrichtung abwechselnd auf beiden Seiten der Klingenebene angeordnet sind.

Es empfiehlt sich, wenn die Führungsmittel in Zuordnung zu jeder der Flachseiten der Schneidklinge mindestens zwei in Klingenlängsrichtung im Abstand voneinander angeordnete Führungsformationen umfassen. Dabei können die Führungsmittel zwei Führungsformationen umfassen, welche bei ordnungsgemäß in die Aufnahme eingesetzter Schneidklingeneinheit in Klingenlängsrichtung beidseits eines Klingenaufsatzes auf einer der Klingenflachseiten angeordnet sind.

Um eine geforderte Flapdicke präzise erzielen zu können, ist es vorteilhaft, die Schneidklinge an ihrem hinteren Klingenrand an einer Führungsanlagefläche des Schneidklingenhalters abgestützt zu führen. Um auch diesen Führungskontakt zwischen Schneidklinge und Schneidklingenhalter reibungsarm und leichtgängig zu gestalten, besitzt die Führungsanlagefläche bei Betrachtung in einem Schnitt quer zur Klingenquerrichtung vorzugsweise eine konvexe, beispielsweise bogenartig gekrümmte Kontur.

Besonders günstig für einen geringen Verschleiß ist es, wenn die Führungsmittel von einem oder mehreren Führungskörpern gebildet sind, welche gesondert von einem Grundkörper des Schneidklingenhalters hergestellt und aus einem härteren Material als dieser gefertigt sind.

Typische Schneidklingen, wie sie zusammen mit einem Schneidklingenhalter der hier betrachteten Art verwendet werden, werden durch Stanzen aus einem Blechmaterial und anschließendes Schleifen zur Bildung der Schneidkante hergestellt. Dabei kann nicht ausgeschlossen werden, dass an den lateralen Randbereichen der Klinge, die ja nicht geschärft werden, feine Grate zurückbleiben. Bewegt sich die Schneidklinge bei ihrer lateralen Oszillation mit ihren lateralen Randbereichen über die Führungsformationen des Schneidklingenhalters, so ist ein Abrieb von feinsten Spänen nicht auszuschließen. Solche Späne können zur Verunreinigungen im Auge führen und sind daher bestmöglich zu vermeiden. Zu diesem Zweck wird empfohlen, dass die Führungsformationen in einem Bereich vorgesehen sind, dessen Erstreckung in Klingenquerrichtung geringer als die einer zur Verwendung mit dem Schneidklingenhalter bestimmten und ausgebildeten Schneidklinge ist. Bei geeigneter Abstimmung des lateralen Oszillationshubs der Schneidklinge und der Quererstreckung der Führungsformationen bzw. des mit solchen Führungsformationen bestückten Bereichs kann dann verhindert werden, dass die Schneidklinge mit ihren lateralen Klingenrändern über die Führungsformationen hinwegfährt. Möglichem Spanabrieb an den lateralen Klingenrändern kann so vorgebeugt werden.

Die Erfindung wird im folgenden anhand von Ausführungsbeispielen weiter erläutert. Es stellen dar:
Figur 1 einen Schnitt durch einen Klingenaufnahmebereich eines Schneidklingenhalters gemäß einem Ausführungsbeispiel,
Figur 2 perspektivisch ein Ausführungsbeispiel einer zur Verwendung mit dem Schneidklingenhalter der Figur 1 geeigneten Schneidklingeneinheit,
Figuren 3 bis 5 Varianten von Führungsmitteln zur Führung einer Schneidklinge der Schneidklingeneinheit der Figur 2,
Figur 6 eine Schnittdarstellung ähnlich Figur 1 bei einem weiteren Ausführungsbeispiel und
Figur 7 eine Perspektivdarstellung eines Teils eines Schneidklingenhalters des Ausführungsbeispiels der Figur 6.

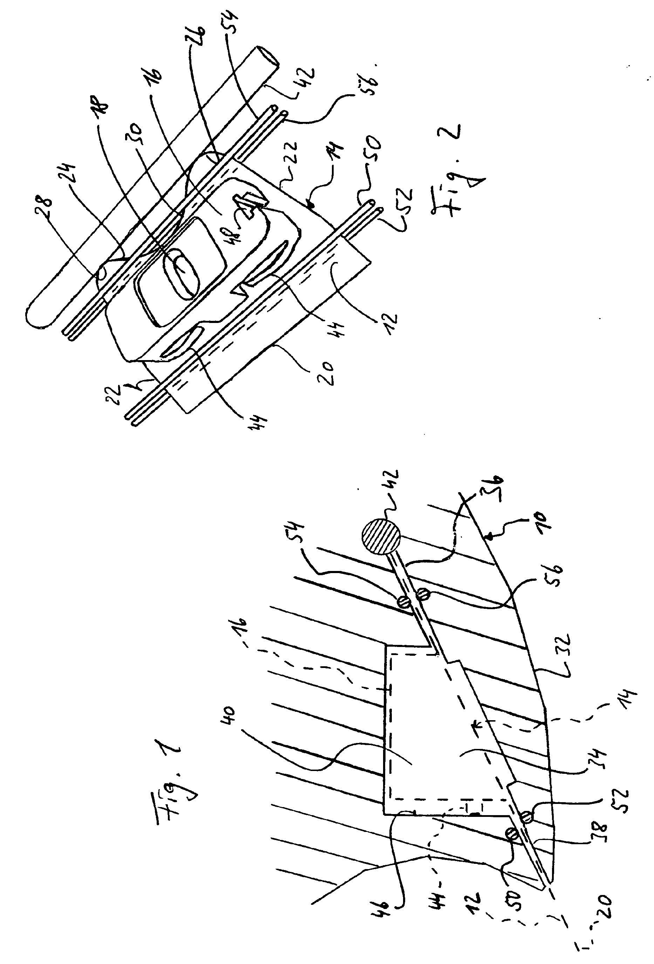

Der in Figur 1 ausschnittsweise gezeigte Schneidklingenhalter - dort allgemein mit 10 bezeichnet - ist in an sich bekannter Weise an einer nicht näher dargestellten Saugringeinheit eines augenchirurgischen Mikrokeratoms beweglich geführt gehalten bzw. halterbar. Mittels eines ebenfalls nicht näher dargestellten elektromotorischen Antriebs ist der Schneidklingenhalter, nachdem die Saugringeinheit auf ein zu operierendes Auge aufgesetzt und dort mittels Vakuum angesaugt wurde, in einer Vorschubrichtung über die Hornhaut des Auges bewegbar, wobei eine Schneidklinge 12 (siehe insbesondere Figur 2) einen oberflächlichen Flap von der Hornhaut abtrennt.

Wie in Figur 2 erkennbar, ist die Schneidklinge 12 Teil einer Schneidklingeneinheit 14, welche zusätzlich zur eigentlichen Schneidklinge 12 einen Aufsatz 16 an einer der Klingenflachseiten umfasst. Der Aufsatz 16 ist fest mit der Schneidklinge 12 verbunden, vorzugsweise durch eine formschlüssige oder kraftschlüssige Verbindung. Auch eine stoffschlüssige Verbindung mittels eines Klebstoffs ist vorstellbar. Der Aufsatz 16 vereinfacht die Handhabung der Schneidklingeneinheit 14. An seiner klingenfernen Oberseite weist er eine längliche Vertiefung 18 auf, in welche im Betrieb des Mikrokeratoms ein Exzenterzapfen einer Abtriebswelle des erwähnten elektromotorischen Antriebs eingreift. Die Schneidklingeneinheit 14 wird bei Rotation der Motorabtriebswelle so in seitlich oszillierende Bewegung (quer zur Vorschubrichtung) versetzt, was die Schneidwirkung verbessert.

Die Schneidklinge 12 bildet an einem geradlinigen vorderen Klingenrand eine Schneidkante 20. Stumpfe seitliche Klingenränder 22 schließen an den vorderen Klingenrand an und gehen im rückwärtigen Bereich der Schneidklinge 12 in einen hinteren Klingenrand 24 über. Der hintere Klingenrand 24 ist mit zwei im Abstand voneinander angeordneten, rundlichen Anlageabschnitten 26, 28 ausgeführt, zwischen denen ein zurückversetzter Klingenrandabschnitt 30 vorhanden ist.

Der Schneidklingenhalter 10 weist einen Grundkörper 32 auf, in dem ein Aufnahmeschacht 34 für die Schneidklingeneinheit 14 ausgebildet ist. Der Aufnahmeschacht 34 ist zu einer lateralen Seite des Schneidklingenhalters 10 hin offen, sodass die Schneidklingeneinheit 14 quer zur Klingenlängsrichtung (die Längsrichtung der Klinge verläuft hierbei zwischen Schneidkante 20 und hinterem Klingenrand 24) in den Aufnahmeschacht 34 eingeschoben und nach Gebrauch wieder herausgezogen werden kann. Der Aufnahmeschacht 34 besitzt zwei schlitzförmige Abschnitte 36, 38, zwischen denen ein aufgeweiteter Abschnitt 40 vorhanden ist. Wird die Schneidklingeneinheit 14 in den Aufnahmeschacht 34 eingesetzt, gelangt der Aufsatz 16 in den aufgeweiteten Abschnitt 40, während die Klingenbereiche vor und hinter dem Aufsatz 16 in die schlitzförmigen Schachtabschnitte 36, 38 eintauchen. Ist die Schneidklingeneinheit 14 ordnungsgemäß in dem Aufnahmeschacht 34 angeordnet, wie gestrichelt in Figur 1 erkennbar, so steht die Schneidklinge 12 mit ihrer Schneidkante 20 aus dem Schneidklingenhalter 10 vor. Gleichzeitig stützt sich die Schneidklinge 12 mit ihren rückwärtigen Randabschnitten 26, 28 an einem in dem Grundkörper 32 gehaltenen Führungsanlagestab 42 ab.

Der Führungsanlagestab 42 ist im Beispielfall der Figuren 1 und 2 mit kreisförmigem Querschnitt ausgeführt und bildet mit seiner Außenumfangsfläche folglich eine konvexe Führungsanlagefläche für die Abschnitte 26, 28 des hinteren Klingenrands 24. Zur Bereitstellung einer konvexen, bogenförmig gekrümmten Führungsanlagefläche kann der Führungsanlagestab 42 auch eine andere Querschnittsform als kreisförmig besitzen, beispielsweise elliptisch oder oval.

Aufgrund der rundlichen Ausbildung der Anlageabschnitte 26, 28 ist der Kontakt zwischen der Schneidklinge und dem Führungsanlagestab 42 nahezu punktuell. Dies sorgt für eine besonders geringe Reibung, wenn der Schneidklingenhalter 14 im Betrieb des Mikrokeratoms seitlich oszilliert. Die geringe Reibung wird durch die konvexe Form der vom Führungsanlagestab 42 gebildeten Führungsanlagefläche noch gefördert. Statt einer bogenförmig gekrümmten Kontur der Führungsanlagefläche ist es sogar vorstellbar, die Führungsanlagefläche bei Betrachtung in einem Schnitt normal zur Klingenquerrichtung mit einer näherungsweise spitz zulaufenden Kontur auszuführen. Auf diese Möglichkeit wird an späterer Stelle noch einmal eingegangen.

In Figur 2 ist zu erkennen, dass der Aufsatz 16 mit zwei Federzungen 44 ausgeführt ist, welche zur Zusammenwirkung mit einer vorderen Begrenzungswand 46 des aufgeweiteten Schachtabschnitts 40 bestimmt und ausgebildet sind. Die Federzungen 44 bewirken eine Vorspannung auf die Schneidklingeneinheit 14 in Richtung nach hinten, d.h. gegen den Führungsanlagestab 42, wenn die Schneidklingeneinheit 14 ordnungsgemäß in den Aufnahmeschacht 34 eingesetzt ist.

Seitlich in den Aufsatz 16, der beispielsweise aus Kunststoff spritzgegossen sein kann, ggf. aber auch aus Metall oder aus einem keramischen Werkstoff gefertigt sein kann, ist eine hinterschnittene T-Nut 48 eingearbeitet, mit welcher eine nicht näher dargestellte Betätigungsstange in schub- und zugkraftübertragenden Eingriff gebracht werden kann. Mittels einer solchen Betätigungsstange kann die Schneidklingeneinheit 14 problemlos in den Aufnahmeschacht 14 eingeschoben und/oder aus diesem herausgezogen werden.

Zur reibungsarmen, gleichzeitig präzisen Führung der Schneidklinge 12 bei deren Oszillationsbewegung dienen bei dem Ausführungsbeispiel der Figuren 1 und 2 zwei Paare von Führungsstäben 50, 52 bzw. 54, 56, welche parallel zum Führungsanlagestab 42, d.h. in Richtung der seitlichen Oszillation der Schneidklingeneinheit 14, in den Grundkörper 32 des Schneidklingenhalters 10 eingebaut sind. Die Führungsstäbe 50 - 56 bilden jeweils eine Führungsformation im Sinne der Erfindung und sind wie der Führungsanlagestab 42 aus einem besonders abriebfesten Material hergestellt, das größere Härte als das Material des Grundkörpers 32 besitzt. Vorzugsweise handelt es sich bei den Stäben 42 und 50 - 56 um Hartmetallstäbe. Der Grundkörper 32 des Schneidklingenhalters kann dagegen beispielsweise aus Edelstahl oder Titan hergestellt sein. Die Führungsstäbe 50 - 56 sind im Bereich der schlitzförmigen Schachtabschnitte 36, 38 angeordnet, nämlich so, dass jedem dieser schlitzförmigen Abschnitte 36, 38 ein Paar einander im wesentlichen gegenüberliegender Stäbe zugeordnet ist. Wenngleich in Figur 1 nicht deutlich erkennbar, ragen die Führungsstäbe 50 - 56 auf einem Teil ihres Umfangs etwas in die schlitzförmigen Schachtabschnitte 36, 38 hinein, sodass die Schneidklinge 12 an ihren Flachseiten allein in Kontakt mit den Außenumfangsflächen der Führungsstäbe 50 - 56 tritt, nicht aber mit den oberen und unteren Begrenzungswänden der schlitzförmigen Schachtabschnitte 36, 38. Der Kontakt zwischen den Führungsstäben 50 - 56 und den Flachseiten der Schneidklinge 12 ist dabei im technischen Sinn linienförmig (im Unterschied zu einer flächigen Anlage) was für eine geringe Reibung sorgt. Wie die Figuren 1 und 2 zeigen, ist ein Paar der Führungsstäbe, nämlich das Paar 54, 56, hinter dem Aufsatz 16 der Schneidklinge 12 angeordnet, während das andere Stabpaar, also das Paar 50, 52, vor dem Aufsatz 16 angeordnet ist. Die Stäbe 50 - 56 und auch der Führungsanlagestab 42 können in entsprechend geformte Kanäle des Grundkörpers 32 eingesetzt sein.

Zur Herstellung des erwähnten Linienkontakts mit der Schneidklinge 12 weisen die Führungsstäbe 50 - 56 konvexe Führungsflächen auf, welche in die schlitzförmigen Schachtabschnitte 36, 38 hineinragen. Im Beispielfall der Figuren 1 und 2 sind die Führungsstäbe 50 - 56 zur Bildung dieser konvexen Führungsflächen mit einem Kreisquerschnitt ausgeführt. Es versteht sich, dass andere Querschnittsformen für die Führungsstäbe 50 - 56 gewählt werden können, sofern sie im Bereich der Anlage der Schneidklinge 12 eine konvexe Kontur besitzen. Beispielsweise kann eine elliptische oder ovale Querschnittsform für die Führungsstäbe 50 - 56 gewählt werden; sie können aber auch eine im Bereich des Kontakts mit der Schneidklinge 12 spitz zulaufende Querschnittsgestalt haben.

Bei einer Abwandlung des in den Figuren 1 und 2 gezeigten Ausführungsbeispiels kann der Grundkörper 32 selbst mit einstückig angeformten Rippen ausgeführt sein, welche die in die schlitzförmigen Schachtabschnitte 36, 38 vorstehenden Segmente der Führungsstäbe 50 - 56 ersetzen. Auch die von dem Führungsanlagestab 42 gebildete konvexe Führungsanlagefläche für den hinteren Klingenrand 24 der Schneidklinge 12 kann bei Bedarf einstückig an dem Grundkörper 32 ausgebildet sein. Die Vorsehung gesonderter Stäbe für die Führung der Schneidklinge 12 ist allerdings insofern von Vorteil, als die Stäbe auswechselbar in den Schneidklingenhalter 10 eingebaut sein können, sodass bei Verschleiß einzelne Stäbe ersetzt werden können.

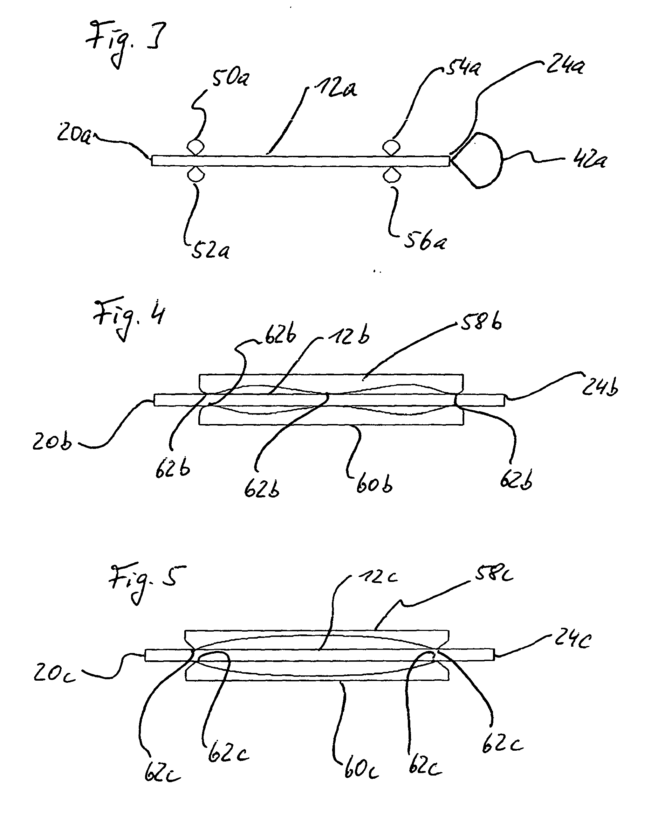

Es wird nun auf die in den Figuren 3 bis 5 schematisch gezeigten Abwandlungen verwiesen. In diesen Figuren sind gleichwirkende Komponenten mit gleichen Bezugszeichen wie zuvor bezeichnet, jedoch ergänzt um einen Kleinbuchstaben.

Bei der Variante der Figur 3 sind die Führungsstäbe 50a - 56a sowie der Führungsanlagestab 42a jeweils mit einer im Bereich des Kontakts mit der Schneidklinge 12a spitz zulaufenden Kontur ausgebildet. Neben der in dieser Figur dargestellten Querschnittsform der Stäbe kann hierzu beispielsweise auch eine dreieckige oder rechteckige Querschnittsform der Stäbe verwendet werden.

Die Figuren 4 und 5 zeigen Varianten, bei denen mehrere Führungsformationen für die Schneidklinge jeweils an einem gemeinsamen Führungskörper ausgebildet sind. In Figur 4 sind zwei Führungsplatten 58b, 60b oberhalb und unterhalb der Schneidklinge 12b angeordnet. Die Führungsplatten 58b, 60b ersetzen die Führungsstäbe 50 - 56 des in den Figuren 1 und 2 gezeigten Ausführungsbeispiels. Sie sind auf ihrer der Schneidklinge 12b zugewandten Plattenseite mit rippenartigen Führungsanlageabschnitten 62b ausgeführt, welche in der Betrachtungsweise der Figur 4, also bei Betrachtung in einem Schnitt quer zur Klingenquerrichtung, rundlich ausgeführt sind. Infolge der rundlichen Kontur der Führungsanlageabschnitte 62b stellt sich wiederum ein näherungsweise linienförmiger Kontakt mit den Flachseiten der Schneidklinge 12b ein. Insgesamt weisen bei der Variante der Figur 4 die beiden Führungsplatten 58b, 60b jeweils drei Führungsanlageabschnitte 62b auf, wobei diese sich einander paarweise gegenüberliegen. Die Führungsplatten 58b, 60b können beispielsweise in geeignete Aussparungen des Grundkörpers des Schneidklingenhalters eingesetzt sein.

Die Variante der Figur 5 unterscheidet sich von derjenigen der Figur 4 dadurch, dass die Führungsplatten 58c, 60c jeweils nur zwei Führungsanlageabschnitte 62c aufweisen und dass diese Führungsanlageabschnitte 62c im Kontaktbereich mit der Schneidklinge 12c eine spitz zulaufende Kontur besitzen.

Es versteht sich, dass auch Ausführungsformen denkbar sind, bei denen auf einer Seite der Schneidklinge ein oder mehrere Führungsstäbe vorgesehen sind, während auf der anderen Seite der Schneidklinge ein Führungskörper vorgesehen ist, der mehrere im Abstand voneinander angeordnete Führungsformationen trägt, wie beispielsweise die Führungsplatten der Figuren 4 und 5.

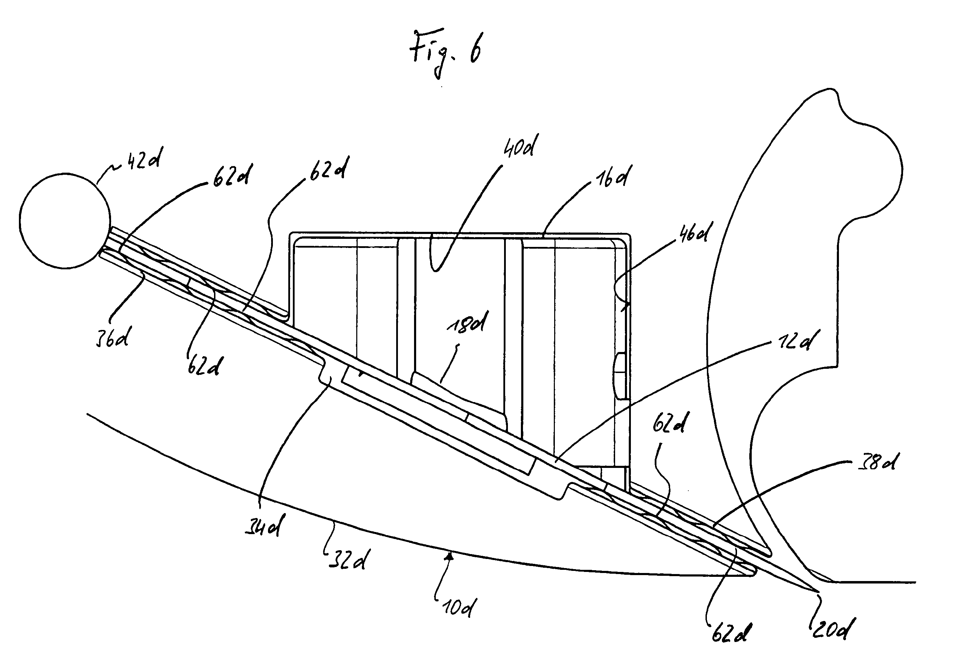

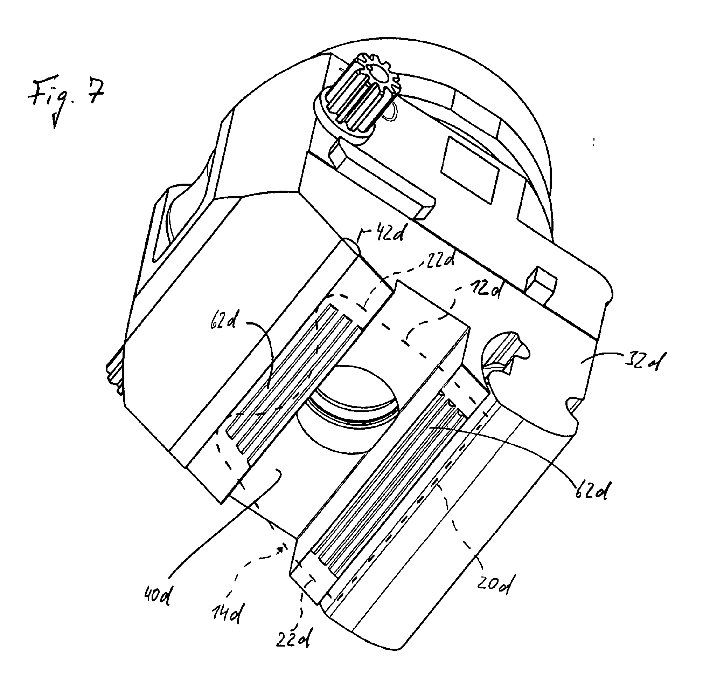

Auch bei dem Ausführungsbeispiel der Figuren 6 und 7 sind gleiche oder gleichwirkende Komponenten wiederum mit gleichen Bezugszahlen wie zuvor bezeichnet, diesmal jedoch ergänzt durch einen Kleinbuchstaben d. Bei diesem Ausführungsbeispiel ist der Schneidklingenhalter 10d beidseits der Klingenebene der Schneidklinge 12d jeweils mit einer Mehrzahl von Führungsrippen 62d versehen, welche parallel zueinander verlaufen und sich in Klingenquerrichtung erstrecken. Die Führungsrippen 62d sind, wie gut in Figur 6 zu erkennen ist, alternierend auf beiden Seiten der Klingenebene angeordnet, nämlich so, dass eine Führungsrippe 62d auf einer Seite der Klingenebene im wesentlichen mittig zwischen zwei Führungsrippen 62d auf der anderen Seite der Klingenebene liegt. Die Führungsrippen 62d können einstückig mit dem Grundkörper 32d des Schneidklingenhalters 10d ausgebildet sein. Alternativ können die Führungsrippen 62d an gesonderten Führungskörpern ausgebildet sein, welche an dem Grundkörper 32d montiert sind. Die Verbindung zwischen diesen Führungskörpern und dem Grundkörper 32d kann unlösbar sein, beispielsweise durch Verklebung oder Einpressen. Es ist aber auch vorstellbar, die Führungskörper auswechselbar an dem Grundkörper 32d anzubringen, sodass ein verschleißbedingter Austausch möglich ist. Wie bereits weiter oben im Zusammenhang mit den Varianten der Figuren 4 und 5 erwähnt, können solche Führungskörper beispielsweise die Form dünner Platten haben, von denen sich die Führungsrippen einstückig abheben.

In Figur 7, in der die Schneidklinge 12d in einer Mittellage gestrichelt eingezeichnet ist, erkennt man, dass die Länge der Führungsrippen 62d geringer ist als die Breite der Schneidklinge 12d. Der laterale Oszillationshub der Schneidklinge 12d ist dabei so eingestellt, dass die seitlichen Klingenränder 22d im Schneidbetrieb der Klinge nicht über die Führungsrippen 62d gelangen. Hierdurch kann ein etwaiger Spanabrieb an den Seiterändern 22d der Klinge vermieden werden.

Der Vollständigkeit halber sei darauf hingewiesen, dass in der Darstellung der Figur 7 der in Figur 6 erkennbare untere Teil des Grundkörpers 32d aus Gründen der Übersichtlichkeit weggelassen ist.

## Patentansprüche

1. Schneidklingenhalter (10) für eine mikrochirurgische Schneidanordnung, insbesondere eine Schneidanordnung für die refraktive Augenchirurgie, wobei der Schneidklingenhalter (10) eine Aufnahme (34) aufweist, in die eine Schneidklingeneinheit (14) mit einer Schneidklinge (12) einsetzbar ist, wobei weiter der Schneidklingenhalter Führungsmittel (42, 50 - 56) zur seitbeweglichen Führung der Schneidklingeneinheit in der Aufnahme umfasst,
**dadurch gekennzeichnet, dass** die Führungsmittel beidseits der Klingenebene der Schneidklinge angeordnete Führungsformationen (50 - 56) umfassen, welche näherungsweise Punkt- oder Linienkontakt mit der Schneidklinge herstellen.

2. Schneidklingenhalter nach Anspruch 1,
**dadurch gekennzeichnet, dass** mindestens eine Führungsformation von einem in Klingenquerrichtung sich erstreckenden, an einem Grundkörper (32) des Schneidklingenhalters (10) gehaltenen Führungsstab (50 - 56) gebildet ist.

3. Schneidklingenhalter nach Anspruch 1,
**dadurch gekennzeichnet, dass** mehrere Führungsformationen (62b) einstückig an einem gemeinsamen Führungskörper (58b, 60b) des Schneidklingenhalters ausgebildet sind.

4. Schneidklingenhalter nach einem der vorhergehenden Ansprüche,
**dadurch gekennzeichnet, dass** mindestens eine Führungsformation (50 - 56) bei Betrachtung in einem Schnitt quer zur Klingenquerrichtung eine bogenartig gekrümmte Kontur im Bereich einer Führungsanlagestelle besitzt.

5. Schneidklingenhalter nach einem der vorhergehenden Ansprüche,
**dadurch gekennzeichnet, dass** mindestens eine Führungsformation (50a - 56a) bei Betrachtung in einem Schnitt quer zur Klingenquerrichtung eine spitz zulaufende Kontur im Bereich einer Führungsanlagestelle besitzt.

6. Schneidklingenhalter nach einem der vorhergehenden Ansprüche,
**dadurch gekennzeichnet, dass** mindestens ein Paar von beidseits der Klingenebene angeordneten Führungsformationen (50 - 56) einander bei Betrachtung in einem Schnitt quer zur Klingenquerrichtung zumindest näherungsweise gegenüberliegt.

7. Schneidklingenhalter nach einem der vorhergehenden Ansprüche,
**dadurch gekennzeichnet, dass** mehrere Führungsformationen (62d) bei Betrachtung in einem Schnitt quer zur Klingenquerrichtung abwechselnd auf beiden Seiten der Klingenebene angeordnet sind.

8. Schneidklingenhalter nach einem der vorhergehenden Ansprüche,
**dadurch gekennzeichnet, dass** die Führungsmittel in Zuordnung zu jeder der Flachseiten der Schneidklinge mindestens zwei in Klingenlängsrichtung im Abstand voneinander angeordnete Führungsformationen (50 - 56) umfassen.

9. Schneidklingenhalter nach Anspruch 8,
**dadurch gekennzeichnet, dass** die Führungsmittel zwei Führungsformationen (50, 54) umfassen, welche bei ordnungsgemäß in die Aufnahme eingesetzter Schneidklingeneinheit (14) in Klingenlängsrichtung beidseits eines Klingenaufsatzes (16) auf einer der Klingenflachseiten angeordnet sind.

10. Schneidklingenhalter nach einem der vorhergehenden Ansprüche,
**dadurch gekennzeichnet, dass** die Führungsformationen (62d) in einem Bereich vorgesehen sind, dessen Erstreckung in Klingenquerrichtung geringer als die einer zur Verwendung mit dem Schneidklingenhalter (10d) bestimmten und ausgebildeten Schneidklinge (12d) ist.

11. Schneidklingenhalter nach einem der vorhergehenden Ansprüche oder nach dem Oberbegriff des Anspruchs 1,
**dadurch gekennzeichnet, dass** die Führungsmittel (42, 50 - 56) eine zur Abstützung der Schneidklinge (14) an einem hinteren Klingenrand (24) derselben bestimmte Führungsanlagefläche umfassen, welche bei Betrachtung in einem Schnitt quer zur Klingenquerrichtung eine konvexe Kontur besitzt.

12. Schneidklingenhalter nach einem der vorhergehenden Ansprüche,
**dadurch gekennzeichnet, dass** die Führungsmittel (42, 50 - 56) von einem oder mehreren Führungskörpern gebildet sind, welche gesondert von einem Grundkörper (32) des Schneidklingenhalters (10) hergestellt und aus einem härteren Material als dieser gefertigt sind.

## Claims

1. A cutting blade holder (10) for a microsurgical cutting apparatus, in particular a cutting apparatus for refracting a surgery, the cutting blade holder (10) including a receptacle (34) into which a cutting blade unit (14) having a cutting blade (12) can be inserted, the cutting blade holder further including guide means (42, 50 - 56) for laterally movably guiding the cutting blade unit in the receptacle,
**characterized in that** the guide means include guide formations (50 - 56) arranged on both sides of the blade plane of the cutting blade, which guide formations establish an approximately punctiform or linear contact with the cutting blade.

2. The cutting blade holder of claim 1,
**characterized in that** at least one guide formation is formed by a guide bar (50 - 56) extending in transverse direction of the blade and supported on a base body (32) of the cutting blade holder (10).

3. The cutting blade holder of claim 1,
**characterized in that** a plurality of guide formations (62b) are formed in one piece on a common guide body (58b, 60b) of the cutting blade holder.

4. The cutting blade holder of anyone preceding claim,
**characterized in that** at least one guide formation (50 - 56) has an arcuately curved contour in the region of a guide contact location, when viewed in a cross-section transverse to the transverse direction of the blade.

5. The cutting blade holder of anyone preceding claim,
**characterized in that** at least one guide formation (50a - 56a) has a pointed contour in the region of a guide contact point, when viewed in a cross-section transverse to the transverse direction of the blade.

6. The cutting blade holder of anyone preceding claim,
**characterized in that** at least one pair of guide formations (50 - 56) arranged on both sides of the plain of the blade are disposed opposite to each other, when viewed in a cross-section transverse to the transverse direction of the blade.

7. The cutting blade holder of anyone preceding claim,
**characterized in that** a plurality of guide formations (62d) are disposed alternately on both sides of the plain of the blade, when viewed in a cross-section transverse to the transverse direction of the blade.

8. The cutting blade holder of anyone preceding claim,
**characterized in that** the guide means include at least two guide formations (50 - 56) in association with each flat surface side of the cutting blade, which guide formations are based apart from each other in the longitudinal direction of the blade.

9. The cutting blade holder of claim 8,
**characterized in that** the guide means include two guide formations (50, 54) arranged at one of the flat surface sides of the blade on both sides of a blade attachment (16) in the longitudinal direction of the blade, when the cutting blade unit (14) is correctly inserted into the receptacle.

10. The cutting blade holder of anyone preceding claim,
**characterized in that** the guide formations (62d) are provided in a region, the extension of which in the transverse direction of the blade is less than that of a cutting blade (12d) designed and intended for use with the cutting blade holder (10d).

11. The cutting blade holder according to anyone preceding claim or according to the preamble portion of claim 1,
**characterized in that** the guide means (42, 50 - 56) include a guide contact surface intended for supporting the cutting blade (14) at a rear blade edge (24) thereof, which guide contact surface has a convex contour when viewed in a cross-section transverse to the transverse direction of the blade.

12. The cutting blade holder of anyone preceding claim,
**characterized in that** the guide means (42, 50 - 56) are formed from one or more guide bodies manufactured separately from a base body (32) of the cutting blade holder (10) and being of a harder material than the base body.

## Revendications

1. Porte-lame (10) pour un dispositif de coupe microchirurgical, en particulier pour la chirurgie réfractive de l'oeil, dans lequel le porte-lame (10) présente un logement (34) dans lequel est mise en place une unité de lame de coupe (14) munie d'une lame de coupe (12), ledit porte-lame comprenant des moyens de guidage (42, 50 à 56) pour le guidage par déplacement latéral de l'unité de lame de coupe dans le logement,
**caractérisé en ce que** les moyens de guidage comportent des formations de guidage (50 à 56) disposées des deux côtés du plan de lame de coupe, par l'intermédiaire desquelles un contact ponctuel ou un contact linéaire est établi approximativement avec la lame de coupe.

2. Porte-lame selon la revendication 1,
**caractérisé en ce qu'**au moins une formation de guidage est constituée par une baguette de guidage (50 à 56) maintenue à un corps de base (32) du porte-lame (10) et s'entendant dans le sens transversal de la lame.

3. Porte-lame selon la revendication 1,
**caractérisé en ce que** plusieurs formations de guidage (62b) sont réalisées d'un seul tenant avec un corps de guidage commun (58b, 60b) du porte-lame.

4. Porte-lame selon l'une des revendications précédentes,
**caractérisé en ce qu'**au moins une formation de guidage (50 à 56), vue en coupe transversalement au sens transversal de la lame, possède un contour courbé en arc dans la zone d'un point d'appui sur le guidage.

5. Porte-lame selon l'une des revendications précédentes,
**caractérisé en ce qu'**au moins une formation de guidage (50a à 56a), vue en coupe transversalement au sens transversal de la lame, possède un contour se terminant en pointe dans la zone d'un point d'appui sur le guidage.

6. Porte-lame selon l'une des revendications précédentes,
**caractérisé en ce qu'**au moins une paire de formations de guidage (50 à 56) disposées des deux côtés du plan de lame de coupe est, vue en coupe transversalement au sens transversal de la lame, située pour le moins approximativement l'une en face de l'autre.

7. Porte-lame selon l'une des revendications précédentes,
**caractérisé en ce que** plusieurs formations de guidage (62d), vues en coupe transversalement au sens transversal de la lame, sont disposées en alternance des deux côtés du plan de lame de coupe.

8. Porte-lame selon l'une des revendications précédentes,
**caractérisé en ce que** les moyens de guidage en association avec chacune des faces plates de la lame de coupe comprennent au moins deux formations de guidage (50 à 56) disposées à distance l'une de l'autre dans le sens longitudinal de la lame.

9. Porte-lame selon la revendication 8,
**caractérisé en ce que** les moyens de guidage comprennent deux formations de guidage (50, 54) qui, lorsque l'unité de lame de coupe (14) est correctement mise en place dans le logement, sont disposées sur une des faces plates de la lame, des deux côtés d'un module à lame (16), dans le sens longitudinal de la lame.

10. Porte-lame selon l'une des revendications précédentes,
**caractérisé en ce que** les formations de guidage (62d) sont prévues dans une zone dont l'étendue dans le sens transversal de la lame est inférieure à celle d'une lame de coupe (12d) réalisée pour et destinée à être utilisée avec le porte-lame (10d).

11. Porte-lame selon l'une des revendications précédentes ou selon le préambule de la revendication 1,
**caractérisé en ce que** les moyens de guidage (42, 50 à 56) comportent une surface d'appui sur le guidage, qui est destinée à supporter la lame de coupe (14) au bord arrière (24) de la lame et qui, vue en coupe transversalement au sens transversal de la lame, possède un contour convexe.

12. Porte-lame selon l'une des revendications précédentes,
**caractérisé en ce que** les moyens de guidage (42, 50 à 56) sont formés par un ou par plusieurs corps de guidage, lesquels sont fabriqués séparément d'un corps de base (32) du porte-lame (10), à partir d'un matériau plus dur que celui du porte-lame.
